(19) Europäisches Patentamt
European Patent Office
Office européen des brevets

(11) Veröffentlichungsnummer: **0 258 172**
B1

# (12) EUROPÄISCHE PATENTSCHRIFT

(45) Veröffentlichungstag der Patentschrift: **08.02.89**

(51) Int. Cl.⁴: **C11B 9/00**, C07C 43/174, A61K 7/46

(21) Anmeldenummer: **87730084.8**

(22) Anmeldetag: **24.07.87**

## (54) Verwendung von Alkylethern des 2,2,2-Trichlor-1-phenylethanols als Riechstoffe.

(30) Priorität: **31.07.86 DE 3626085**

(43) Veröffentlichungstag der Anmeldung: **02.03.88 Patentblatt 88/9**

(45) Bekanntmachung des Hinweises auf die Patenterteilung: **08.02.89 Patentblatt 89/6**

(84) Benannte Vertragsstaaten: **CH DE ES FR GB LI NL**

(56) Entgegenhaltungen: **S. ARCTANDER: "Perfume and Flavor Chemicals", 1969, Montclair, N.J., US**

(73) Patentinhaber: **Dragoco Gerberding & Co. GmbH, Dragocostrasse 1, D-3450 Holzminden(DE)**

(72) Erfinder: **Brunke, Ernst-Joachim, Dr., Dipl.-Chem., Holbeinstrasse 6, D-3450 Holzminden(DE)**
Erfinder: **Kappey, Claus-Hermann, Dr., Dipl.-Chem., Schratweg 1, D-3450 Holzminden(DE)**

(74) Vertreter: **Eikenberg & Brümmerstedt Patentanwälte, Schackstrasse 1, D-3000 Hannover 1(DE)**

**EP 0 258 172 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Seit langem ist bekannt (Steffen Arctander, "Perfume and Flavor Chemicals", Vol. 2, Montclair, N.J., USA 1969, Ref. No. 2976–2978), daß Carbonsäureester des 2, 2, 2-Trichlor-1-phenylethanols, wie das 2, 2, 2-Trichlor-1-phenylethylacetat, -propionat und -butyrat olfaktorische Eigenschaften aufweisen und als Bestandteile von Parfümölen eingesetzt werden können. Dies gilt insbesondere für das Acetat, welches die Formel B

B

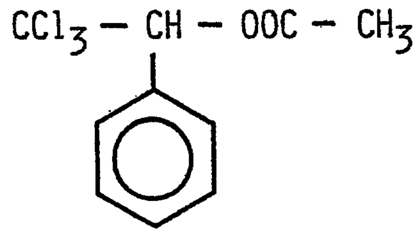

besitzt, verhältnismäßig einfach herstellbar ist und sich gemäß Arctander durch einen milden balsamartigen, grün-rosigen Duft von guter Haftung auszeichnet. Allerdings sind diese Ester nur in kleinen Mengen verwendbar, weil der Duft auch unerwünschte erdige Nebennoten enthält, die bei höheren Konzentrationen störend in Erscheinung treten. Außerdem ist die Stabilität dieser Ester unbefriedigend, insbesondere beim Einsatz in Waschmitteln und ähnlichen Produkten mit basischem Milieu.

Somit besteht ein erheblicher Bedarf an synthetischen rosenartigen Riechstoffen, die sich günstig und mit gleichbleibender Qualität herstellen lassen, bei längerer Lagerung auch im Kontakt mit anderen Stoffen, insbesondere im basischen Milieu, stabil bleiben und angenehme, möglichst naturnahe Duftnoten von ausreichender Intensität aufweisen, um den Duft von kosmetischen oder technischen Konsumgütern vorteilhaft beeinflussen zu können.

Es wurde nun gefunden, daß Alkylether des 2, 2, 2-Trichlor-1-phenylethanols mit der allgemeinen Formel A

A

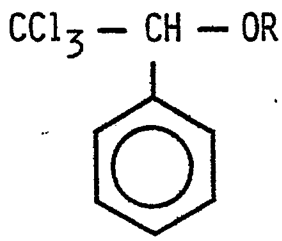

$R = C_1 - C_5$ - Alkyl

diese Forderung in nahezu idealer Weise erfüllen, denn sie sind problemlos herstellbare, sehr stabile Verbindungen mit äußerst vorteilhaften olfaktorischen Eigenschaften. Sie besitzen nämlich naturnahe Duftnoten vom allgemeinen Typ "blumig-rosig" mit würzigen Nebenaspekten, die gute Haftung mit großer Ausstrahlung verbinden und auch in größerer Konzentration keine störenden, z.B. erdigen Nebennoten enthalten. Außerdem sind sie als Bestandteile von Parfumölen, insbesondere solche vom Typ Rose, ganz hervorragend geeignet, indem sie deren Frische, Haftung und Ausstrahlung beträchtlich verstärken.

Demgemäß bezieht sich die Erfindung auf die Verwendung von Alkylethern des 2, 2, 2-Trichlor-1-phenylethanols der allgemeinen Formel A als Riechstoffe, insbesondere als Bestandteile von Parfümölen für kosmetische und technische Konsumgüter. Im einzelnen umfaßt die Formel A dabei Ether mit folgenden Alkylresten:

R = Methyl
R = Ethyl
R = Propyl bzw. Isopropyl
R = Butyl bzw. dessen Isomere und
R = Amyl (Pentyl) bzw. dessen Isomere.

Aus dieser Gruppe ist der Methylether die bevorzugte Verbindung. Sein Duft hat große Ausstrahlung und weist eine rosige Hauptnote mit würzig-holzigen Nebenaspekten auf, was stark gefragt und sehr erwünscht ist. Auch bei den höheren Ethern ist eine rosige Hauptnote vorhanden, jedoch verschieben sich die Nebenaspekte mehr in Richtung würzig-krautig. Damit sind auch die höheren Ether sehr interessant.

Der Methylether ist in anderem Zusammenhang und ohne Hinweis auf irgendwelche olfaktorischen Eigenschaften bereits in der Literatur beschrieben (Chem. Ber. 110, S. 96–106 (1977) sowie Angew. Chemie 85, S. 868–869 (1973)), während die übrigen Ether neue Verbindungen darstellen. Die vorteilhaften olfaktorischen Eigenschaften aller dieser Ether sind überraschend und unterscheiden sich deutlich von denen der eingangs beschriebenen Carbonsäureester des 2, 2, 2-Trichlor-1-phenylethanols. Sie waren auch nicht aus dem olfaktorischen Verhalten strukturverwandter Verbindungen ableitbar, denn außer den Carbonsäureestern, die bereits seit dem Ende des vergangenen Jahrhunderts im Einsatz sind, war bislang in dieser Hinsicht nur noch der zugrundeliegende Alkohol 2, 2, 2-Trichlor-1-phenylethanol untersucht worden, der nicht zum Grundtyp "Rose" gehört und als Riechstoff keine Bedeutung hat (Arctander, loc. cit., Ref. No. 2975). Abgesehen davon gilt auch generell, daß die olfaktorischen Eigenschaften bekannter Riechstoffe keine zwingenden Rückschlüsse auf die Eigenschaften strukturverwandter Verbindungen zulassen, weil weder der Mechanismus der Duftwahrnehmung noch der Einfluß der chemischen Struktur auf die Duftwahrnehmung hinreichend erforscht sind, somit also nicht vorhergesehen werden kann, ob ein abgeänderter Aufbau bekannter Riechstoffe überhaupt zu Änderungen der olfaktorischen Eigenschaft führt und ob diese Änderungen positiv oder negativ beurteilt werden.

Zur Herstellung der Alkylether gemäß der allgemeinen Formel A kann nach den üblichen bekannten Methoden der Etherherstellung vorgegangen werden, beispielsweise durch Umsetzung des 2, 2, 2-Trichlor-1-phenylethanols unter Verwendung von Alkalihydrid und den dem Rest R entsprechenden Alkylsulfaten, Alkylhalogeniden oder Alkyltosylaten, bzw. unter Phasentransfer-Bedingungen mit Alkalihydroxid. (Chem. Ber. 110, loc. cit. sowie Angew. Chemie 85. loc. cit.).

Die nachfolgenden Verwendungsbeispiele sollen die Erfindung erläutern ohne sie einzuschränken. Mit "GT" sind dabei jeweils Gewichtsteile bezeichnet.

Beispiel 1

Ausgegangen wurde von einer Rosen-Base mit der Zusammensetzung:

**Beispiel 1**

| Ausgegangen wurde von einer Rosen-Base mit der Zusammensetzung: | |
|---|---|
| 10 GT | Brahmanol® |
| 40 GT | Phenylethylalkohol |
| 140 GT | Citronellol |
| 100 GT | Geraniol/Nerol |
| 6 GT | Linalool |
| 4 GT | Farnesol |
| 4 GT | Aldehyd C-9 (10%ig in Dipropylenglykol) |
| 8 GT | Phenylacetaldehyd 50%ig |
| 5 GT | Citral |
| 2 GT | (+)-Carvon |
| 15 GT | Citronellylacetat |
| 2 GT | Phenylessigsäuremethylester |
| 10 GT | Methyleugenol |
| 1 GT | Rosenoxid inaktiv |
| 3 GT | Isodamascon (10%ig in Dipropylenglycol) |
| 350 GT | |

Diese Rosen-Base besteht im wesentlichen aus den Hauptriechstoffen des Rosenöls und besitzt einen süßen Rosen-Akkord. Durch Zugabe von 150 GT des Methylethers gemäß Formel A entsteht eine andere Komposition vom Typ "Rose", die sich bei einem relativ hohen Gehalt von 30 Gew.-% an Methylether durch eine natürliche Frische und große Ausstrahlung auszeichnet.

Beispiel 2

Ausgegangen wurde von einer Rosen-Base der Zusammensetzung:

**Beispiel 2**

**Ausgegangen wurde von einer Rosen-Base mit der Zusammensetzung:**

| | |
|---|---|
| 5 GT | Aldehyd C-9 (10%ig in Dipropylenglycol) |
| 5 GT | Isocyclocitral |
| 5 GT | Isodamascon |
| 10 GT | Rosenoxid links |
| 10 GT | Eugenol |
| 20 GT | Dimethylbenzylcarbinylacetat |
| 20 GT | Phenylacetaldehyddimethylacetal |
| 250 GT | Rhodinol |
| 600 GT | Phenylethylalkohol |
| 925 GT | |

Diese Rosen-Base ist im wesentlichen durch die relativ hohe Flüchtigkeit ihrer Rosennote gekennzeichnet. Der Zusatz von 75 GT des Methylethers gemäß Formel A (7,5 Gew.-% an Methylether im resultierenden Parfümöl) führt zu einer deutlich verbesserten Haftung dieser Geruchsnote und verstärkt gleichzeitig den Rosenblütenaspekt in vorteilhafter Weise.

**Patentansprüche**

Verwendung von Alkylethern des 2, 2, 2-Trichlor-1-phenylethanols mit der allgemeinen Formel A

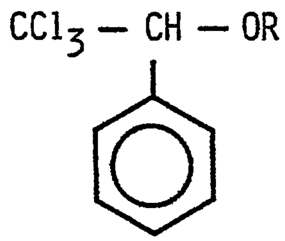

A

R = $C_1$ - $C_5$ - Alkyl

als Riechstoffe, insbesondere als Bestandteile von Parfümölen für kosmetische und technische Konsumgüter.

**Claims**

Use of 2, 2, 2-trichloro-1-phenylethanol alkyl ethers having the general formula A

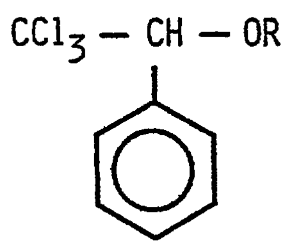

A

R = $C_1$ - $C_5$ - Alkyl

as scents, particularly as components of perfume oils for cosmetic and technical consumer goods.

**Revendications**

Utilisation d'éthers alcoyliques de 2, 2, 2-trichloro-1-phényléthanol de formule générale A

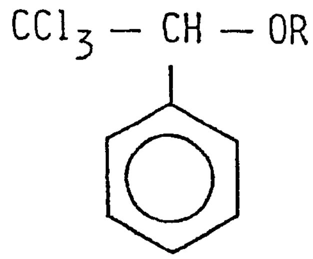

A

$R = C_1 - C_5$ - ALCOYLE

comme substances parfumantes, notamment composants d'essences de parfum pour des produits de consommation cosmétiques et techniques.